(19) Europäisches Patentamt
European Patent Office
Office européen des brevets

(11) Veröffentlichungsnummer: **0 090 353**
**A1**

(12) # EUROPÄISCHE PATENTANMELDUNG

(21) Anmeldenummer: **83102926.9**

(22) Anmeldetag: **24.03.83**

(51) Int. Cl.³: **C 07 D 213/64**, A 61 K 31/44, C 07 D 409/04 // (C07D409/04, 333/00, 213/00)

(30) Priorität: **27.03.82 DE 3211395**

(43) Veröffentlichungstag der Anmeldung: **05.10.83 Patentblatt 83/40**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Müller, Werner Heinrich, Dr., Taunusblick 5, D-6239 Eppstein/Taunus (DE)**
Erfinder: **Blume, Ernst, Dr., Im Hainpfad 10, D-6232 Bad Soden am Taunus (DE)**
Erfinder: **Schleyerbach, Rudolf, Dr., Finkenweg 10, D-6238 Hofheim am Taunus (DE)**

(54) **Pyridon-2-derivate, Verfahren zu ihrer Herstellung und sie enthaltende Arzneimittel.**

(57) Es werden Pyridon-2-derivate der Formel

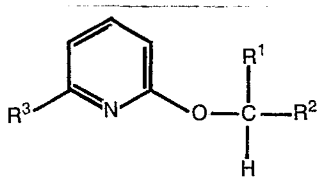

ihre Stereoisomeren und ihre Salze mit einer physiologisch verträglichen Säure oder Base beschrieben, sowie Verfahren zu ihrer Herstellung, in der
$R^1$ Wasserstoff oder Alkyl
$R^2$ eine $-CH=O$, $-COOR^4$ oder $-CH_2-O-R^5$-Gruppe bedeutet, wobei
$R^4$ für Wasserstoff, Alkylatomen oder Benzyl und
$R^5$ für Wasserstoff, Alkyl, Alkanoyl, Cycloalkanoyl, Alkoxycarbonyl, Phenoxycarbonyl oder eine Benzoylgruppe stehen, welche ein oder zwei Halogen-, Trifluormethyl- oder Alkyl- oder Alkoxy-Substituenten tragen kann und
$R^3$ für 2-Thienyl, 3-Thienyl, tert.-Butyl oder eine Phenylgruppe steht, welche einen oder zwei Substituenten tragen kann, die Halogen, Trifluormethyl oder Alkyl oder Alkoxy bedeuten.

Die Verbindungen haben entzündungshemmende Wirkungen und können als Antiphlogistica verwendet werden.

ACTORUM AG

Pyridon-2-derivate, Verfahren zu ihrer Herstellung und sie enthaltende Arzneimittel

Gegenstand der Erfindung sind Pyridon-2-derivate, Verfahren zu ihrer Herstellung und Pyridonderivate enthaltende Arzneimittel mit entzündungshemmender Wirkung.

Bekannte entzündungshemmende Mittel sind mit erheblichen Nebenwirkungen behaftet, die sich insbesondere bei längerer Behandlung störend bemerkbar machen. Es besteht daher ein Bedürfnis an Verbindungen mit antiphlogistischer Wirkung, die eine verbesserte gastrale Verträglichkeit zeigen.

Gegenstand der Erfindung sind Pyridon-2-derivate der Formel I,

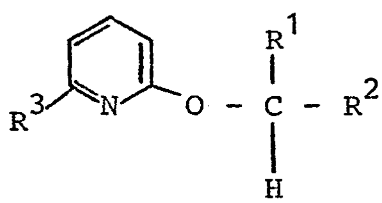 (I)

ihre Stereoisomeren und ihre Salze mit einer physiologisch verträglichen Säure oder, falls $R^2$ eine Carboxylgruppe darstellt, mit einer Base, in der
$R^1$ Wasserstoff oder Alkyl mit 1 bis 6 Kohlenstoffatomen,

$R^2$ eine $-CH{=}O$, $-COOR^4$ oder $-CH_2-O-R^5$-Gruppe bedeutet, wobei
$R^4$ für Wasserstoff, Alkyl mit 1 bis 12 Kohlenstoffatomen oder Benzyl und
$R^5$ für Wasserstoff, Alkyl mit 1 bis 4 Kohlenstoffatomen, Alkanoyl mit 1 bis 9 Kohlenstoffatomen, Cycloalkanoyl mit 4 bis 7 Kohlenstoffatomen, Alkoxycarbonyl mit 2 bis 5 Kohlenstoffatomen, Phenoxycarbonyl

oder eine Benzoylgruppe stehen, welche ein oder zwei Halogen-, Trifluormethyl- oder Alkyl- oder Alkoxy-Substituenten mit jeweils 1 bis 4 Kohlenstoffatomen tragen kann und

$R^3$ für 2-Thienyl, 3-Thienyl, tert.-Butyl oder eine Phenylgruppe steht, welche einen oder zwei Substituenten tragen kann, die Halogen, Trifluormethyl oder Alkyl oder Alkoxy mit jeweils 1 bis 4 Kohlenstoffatomen bedeuten.

Bevorzugt sind Verbindungen der Formel I, in denen $R^1$ für Methyl, Ethyl oder Propyl steht und $R^2$ eine $-CH{=}O$, $-COOCH_3$, $-COOC_2H_5$, $-COOH$ oder eine $-CH_2-O-R^5$ -Gruppe bedeutet. Dabei bedeutet $R^5$ Wasserstoff, eine Formyl-, Acetyl-, Propanoyl-, n-Butanoyl-, 2,2-Dimethylpropanoyl-, n-Octanoyl-, Cyclopropanoyl-, Cyclobutanoyl-, Cyclopentanoyl- oder Cyclohexanoylgruppe, eine Methoxy- oder Ethoxycarbonyl- oder eine Benzoylgruppe, wobei letztere entweder unsubstituiert ist oder einfach mit Fluor, Chlor, Brom, Trifluormethyl oder Methyl substituiert ist.

Für $R^3$ kommt neben der 2-Thienyl-, 3-Thienyl- und tert.-Butylgruppe vor allem die Phenylgruppe in Betracht, die entweder unsubstituiert ist oder einen Substituenten trägt, wobei Fluor, Chlor, Brom, Trifluormethyl, Methyl und Methoxy als Substituenten bevorzugt sind.

Besonders hervorzuheben sind Verbindungen der Formel I, in denen $R^1$ für Methyl oder Ethyl steht, in denen $R^2$ $-CH{=}O$, $-COOCH_3$, $-COOC_2H_5$, $-COOH$ oder eine $-CH_2-O-R^5$-Gruppe ist, wobei $R^5$ Wasserstoff, Acetyl, Propanoyl, 2-Methylpropanoyl oder n-Butanoyl bedeutet und $R^3$ 2-Thienyl-, Phenyl, 4-Fluorphenyl oder tert.-Butyl ist.

Ganz besonders bevorzugt sind Verbindungen der Formel I, in denen $R^1$ für Methyl und $R^2$ für eine $-COOCH_3$-, -COOH- oder eine $-CH_2OH$-Gruppe steht und $R^3$ 2-Thienyl, vor allem Phenyl oder 4-Fluorphenyl bedeutet.

Die Verbindungen der allgemeinen Formel (I) weisen ihre wesentlichen Eigenschaften auch in Form ihrer Salze auf. Zur Herstellung von Säureadditionssalzen kommen alle physiologisch verträglichen Säuren in Frage. Hierzu gehören vorzugsweise die Halogenwasserstoffsäuren, wie z.B. Chlorwasserstoff- und Bromwasserstoff-säure, sowie Salpetersäure, ferner Phosphorsäure oder Schwefelsäure. Bevorzugte organische Säuren sind mono- und bifunktionelle Carbonsäuren und Hydroxycarbonsäuren wie z.B. Essigsäure, Maleinsäure, Oxalsäure, Bernsteinsäure, Fumarsäure, Weinsäure, Zitronensäure, Salicylsäure, Sorbinsäure, Milchsäure sowie Sulfonsäuren wie z.B. p-Toluolsulfonsäure, Methyl- und Phenylsulfonsäure sowie 1,5-Naphthalindisulfonsäure.
Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure oder Salpetersäure,erhalten werden und in bekannter Weise, z.B. durch Abfiltrieren, isoliert und gegebenenfalls durch Waschen oder Umkristallisation mit einem inerten organischen Lösungsmittel gereinigt werden.

Die Salze der Verbindungen der Formel I mit physiologisch verträglichen Basen sind zweckmäßig Natrium-, Kalium-, Magnesium- oder Calcium-Salze derjenigen Verbindungen der Formel I, in denen $R^2$ eine Carboxylgruppe bedeutet.

Gegenstand der Erfindung ist auch ein Verfahren zur Herstellung der Verbindungen der Formel I, das dadurch gekennzeichnet ist, daß man eine Verbindung der Formel II

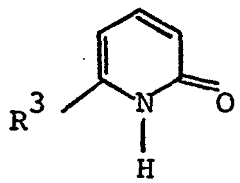

(II)

mit einer Verbindung der Formel III

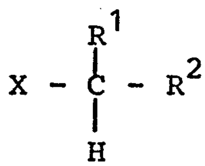

(III)

in denen $R^1$, $R^2$ und $R^3$ die oben angegebenen Bedeutungen haben und X für Halogen, Alkylsulfonyloxy, substituiertes oder unsubstituiertes Phenylsulfonyloxy oder den Rest eines aktiven Esters steht, umsetzt. Insbesondere bedeutet X hier Chlor, Brom, Methylsulfonyloxy, Phenyl-, 4-Methylphenyl- oder 4-Chlorphenylsulfonyloxy oder den Rest eines aktiven Esters wie Trifluoracetyloxy, Phenylcarbonyloxy oder Methoxycarbonyloxy.

Das Verfahren gemäß der Erfindung wird zwischen 30 und 150°C, vorzugsweise zwischen 40 und 120°C, in Anwesenheit einer Base in Wasser oder in einem inerten organischen Lösungsmittel wie N,N-Dimethylformamid, N,N-Dimethylacetamid, Dimethylsulfoxid, Acetonitril, N-Methylpyrrolidon-2, Dioxan, Tetrahydrofuran, Ether, 4-Methyl-2-pentanon, Methanol, Ethanol, Isopropylalkohol, Propanol, Butanol Pentanol, tert.-Butylalkohol, Methylglykol, Dimethylglykol, Dimethyldiglykol, Methyl-butyl-diglykol oder in Polyethylenglykolen durchgeführt. Außerdem können auch Methylenchlorid oder ein aromatischer Kohlenwasserstoff wie Benzol, Chlorbenzol, Toluol oder Xylol sowie Gemische der vorstehend genannten Lösungsmittel verwendet werden.

Geeignete Basen sind beispielsweise Alkalimetall- oder Erdalkalimetall-carbonate, -hydrogencarbonate, -hydroxide, -alkoholate oder -hydride wie Natriumcarbonat, Natriumhydrogencarbonat, Kaliumcarbonat, Natriumhydroxid, Natriummethylat oder Natriumhydrid. Auch organische Basen wie Triethylamin, Tributylamin, Ethylmorpholin, Pyridin, Dimethylaminopyridin oder Chinolin können hier eingesetzt werden.

Die Umsetzung verläuft unter guten Ausbeuten auch unter den Bedingungen einer Phasentransferreaktion, indem man die Reaktionspartner in einem der vorstehenden Lösungsmittel unter kräftigem Rühren in Gegenwart eines Phasentransferkatalysators und entweder einem gepulverten Alkalihydroxid oder einer konzentrierten wäßrigen Lösung eines Alkalihydroxids in einem Temperaturbereich zwischen 20 und 150°C aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise bis zu 12 Kohlenstoffatomen im Alkylrest oder Kronenether wie 12-Crown-4, 15-Crown 5, 18-Crown-6 oder Dibenzo-18-crown-6.

Zur Herstellung einer Verbindung der Formel I, in der $R^2$ eine $-CH_2-O-R^5$-Gruppe ist, geht man zweckmäßigerweise so vor, daß eine Verbindung der Formel I, in der $R^2$ eine $-COOR^4$-Gruppe ist, zur $CH_2OH$-Gruppe reduziert wird, welche dann mit Alkylierungs- oder Acylierungsmitteln zur $-CH_2-O-R^5$ Gruppe umgesetzt wird. Als Reduktionsmittel eignen sich z.B. Lithiumaluminiumhydrid, Diisobutylaluminiumhydrid oder Natriumborhydrid, die in einem inerten Lösungsmittel bei Temperaturen zwischen 0 und 80°C eingesetzt werden.

Die Alkylierungen und Acylierungen werden in einem inerten Lösungsmittel in Gegenwart einer geeigneten Base und bei

Temperaturen zwischen -10 bis 120°C durchgeführt.

Die Herstellung von Verbindungen der Formel I, in denen $R^2$ eine -CH=O-Gruppe ist, erfolgt zweckmäßigerweise durch Oxidation von Verbindungen der Formel I, in denen $R^2$ eine $CH_2OH$-Gruppe ist. Geeignete Oxidationsmittel hierfür sind Bleitetraacetat, Chromtrioxid, Chrom-6-oxid-Bis-pyridin-Komplex, Mangandioxid, $Ni_2O_3 \cdot 3H_2O$, Dimethylsulfoxid / Trifluoressigsäureanhydrid, Dimethylsulfoxid / Oxalylchlorid, Dimethylsulfoxid / Cyclohexylcarbodiimid / Phosphorsäure, Pyridin / $SO_3$-Komplex oder Aluminiumisopropanolat in Kombination mit einer Carbonylverbindung wie 4-Methoxybenzaldehyd, 2-Chlorbenzaldehyd, Zimtaldehyd oder Benzophenon. Als Lösungsmittel werden außer denjenigen, die vorstehend für die Umsetzung der Verbindung II mit der Verbindung III genannt wurden, auch noch Eisessig, Chloroform, Tetrachlorkohlenstoff und Petroläther verwendet. Doch auch eine katalytische Oxidation mit Hilfe eines ein Edelmetall der VIII.Gruppe, insbesondere Platin und/oder Palladium enthaltenden Katalysators ist möglich.

Die als Zwischenprodukte benötigten Verbindungen der Formel II können entweder entsprechend dem von J.Thesing et al., Chem.Ber. 90, 711 (1957) angegebenen Verfahren oder in Analogie zu den von F.Kröhnke et al., in Chem. Ber. 103, 322 ff (1970) angegebenen Vorschriften hergestellt werden. Besonders vorteilhaft ist es jedoch, eine Verbindung der Formel IV

$$R^3 - \underset{\underset{O}{\|}}{C} - CH_3 \qquad (IV)$$

mit einem Acrylsäureester der Formel V

$$CH_2 = CH - \underset{\underset{O}{\|}}{C} - OR^6 \qquad (V)$$

zu einer Verbindung der Formel VI

$$R^3-\underset{\underset{O}{\|}}{C}-CH_2-CH_2-CH_2-\underset{\underset{O}{\|}}{C}-OR^6 \qquad (VI)$$

umzusetzen, welche dann mit Ammoniak zu einer Verbindung der Formel VII

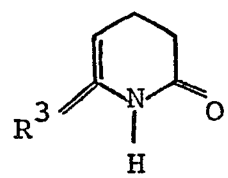

(VII)

cyclisiert und schließlich zu einer Verbindung der Formel II dehydriert wird. In den vorstehend genannten Formeln hat $R^3$ die in Anspruch 1 genannten Bedeutungen und $R^6$ ist ein Alkylrest mit 1 bis 6 Kohlenstoffatomen.

Die Herstellung der Verbindung VI aus dem Keton IV und dem Acrylsäureester V wird bei Temperaturen zwischen 20 und 220°C unter alkalischen Bedingungen in einem inerten Lösungsmittel durchgeführt. Es ist auch möglich, bei dieser Reaktion auf die Verwendung von Lösungsmitteln zu verzichten und die Umsetzung in Substanz durchzuführen. Als Base wird der Reaktionsmischung vorzugsweise ein primäres Amin wie Methyl-, Ethyl-, Propyl-, Isopropyl-, Butyl-, Cyclohexylamin oder das aus dem verwendeten Acrylester und Amin gebildete ß-N-Alkylaminopropionat zugesetzt. Die Reaktion erfolgt in Gegenwart katalytischer Mengen einer Säure wie Benzoesäure, Phenylsulfonsäure, Toluolsulfonsäure, Salicylsäure, Essigsäure oder Acrylsäure.

Die Verbindung VI wird dann mit Ammoniak in einem inerten Lösungsmittel oder in Substanz bei einer Temperatur zwischen 100 und 250°C, vorzugsweise in einem Autoklaven

zur Verbindung VII umgesetzt. Diese wird anschließend mit einem Dehydrierungskatalysator wie Palladium auf Kohle zu der Verbindung II dehydriert.

Die als Ausgangsstoffe verwendeten Verbindungen der Formel III sind bekannt.

Aus der Formel I ist ersichtlich, daß die erfindungsgemäßen Verbindungen mindestens ein asymmetrisches Zentrum in α-Stellung zum Sauerstoffatom aufweisen, sofern $R^1$ nicht Wasserstoff bedeutet. Die bei Verwendung von racemischen Verbindungen der Formel III erhältlichen Racemate der Formel I können in üblicher Weise in ihre optischen Antipoden getrennt werden. So können Verbindungen der Formel I, in denen $R^2$-COOH bedeutet, mit Hilfe einer optisch aktiven Base behandelt werden. Vorteilhaft erhält man die optischen Antipoden von Verbindungen der Formel I, wenn schon die Ausgangsstoffe der Formel III optisch aktiv sind.

Aufgrund der folgenden pharmakologischen Prüfungen und der dabei erhaltenen Ergebnisse können die erfindungsgemäßen Verbindungen als Substanzen mit ausgeprägt entzündungshemmender Wirkung bei gleichzeitig niedriger Toxizität bezeichnet werden. Sie sind deshalb als Arzneimittel mit antiphlogistischer Wirkung, insbesondere gegen rheumatische Erkrankungen verwendbar.

1. Adjuvans Arthritis

Die Untersuchungen wurden nach der Methode von Pearson durchgeführt (Arthrit. Rheum. 2, 440 (1959)). Als Versuchstiere dienten männliche Ratten eines Wistar-Lewis-Stammes im Körpergewicht zwischen 130 und 200 g. Die zu vergleichenden Verbindungen wurden täglich vom 1. bis zum 5. Versuchstag oral appliziert. Tiere einer

Kontrollgruppe erhielten nur das Lösungsmittel. Pro Dosierung und in der Kontrollgruppe wurden jeweils 8 Tiere verwendet. Als Wirkungskriterium diente die Herabsetzung der Pfotenvolumenzunahme gegenüber der unbehandelten Kontrollgruppe. Die $ED_{50}$-Werte wurden graphisch aus der Dosiswirkungskurve bestimmt.

2. Gastrale ulzerogene Wirkung

Die Untersuchungen erfolgten an jeweils 10 männlichen Sprague-Dawley Ratten im Körpergewicht von 200-300 g. 48 Stunden vor Applikation der zu vergleichenden Verbindungen bis zum Töten der Tiere wurde das Futter entzogen, bei freiem Zugang zum Trinkwasser. Die Ratten wurden 24 Stunden nach oraler Gabe getötet, der Magen entnommen, unter fließendem Wasser gereinigt und auf Schleimhautläsionen inspiziert. Als Ulzera galten alle makroskopisch sichtbaren Läsionen. Bestimmt wurde der Anteil der Tiere mit Ulzera pro dosi und die $UD_{50}$ nach Lichtfield und Wilcoxon (J. Pharmacol. exp. Ther. 96, 99 (1949)).

3. Akute Toxizität

Die Bestimmung der akuten Toxizitäten erfolgten in Standardmethoden an Ratten des Sprague-Dawley-Stammes. Pro Dosierung wurden 6 Tiere verwendet. Die $LD_{50}$-Werte wurden nach Lichtfield und Wilcoxon bestimmt.

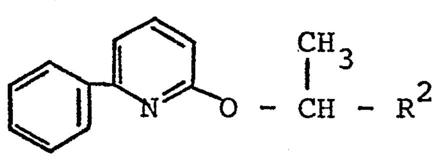

| Substanz | $R^2$ | $ED_{50}$ Adjuvans-Arthritis | $UD_{50}$ gastrale Ulcera | $TI (UD_{50}/ED_{50})$ therapeutischer Index |
|---|---|---|---|---|
| Sulindac | | 13.0 mg/kg | 26.0 mg/kg | 2 |
| Naproxen | | 15.0 mg/kg | 21.0 mg/kg | 2 |
| Beisp. 4 | COOH | 20,0 mg/kg | 175 mg/kg | 9 |
| Beisp. 7 | $COOC_2H_5$ | 15.0 mg/kg | 125 mg/kg | 8 |
| Beisp. 9 | $CH_2OH$ | 20.0 mg/kg | 220.0 mg/kg | 11 |

| Substanz | $LD_{50}$ akute Toxizität | $TI (LD_{50}/ED_{50})$ therapeutischer Index |
|---|---|---|
| Naproxen | 395 mg/kg | 26 |
| Beispiel 9 | 1000 mg/kg | 50 |

Die untersuchten Verbindungen zeigten einen eindeutigen Vorteil gegenüber den bekannten Vergleichspräparaten Sulindac und Naproxen aufgrund ihrer ausgezeichneten gastralen Verträglichkeit an der Ratte, einem Tiermodell mit guter Aussagekraft über die wichtigste Nebenwirkung dieser Substanzklasse beim Menschen. Daraus leitet sich ein therapeutischer Index ab, der mindestens das 4-fache der Vergleichspräparate beträgt. Der Alkohol (Beispiel 9) weist darüberhinaus eine für Antiphlogistika ungewöhnlich gute akute Toxizität auf ($LD_{50}$ 1000 mg/kg), die mehr als das zweifache der $LD_{50}$ von Naproxen beträgt (395 mg/kg).

Die erfindungsgemäßen Arzneimittel können oral, rektal oder parenteral verabreicht werden.

Geeignete feste oder flüssige galenische Zubereitungsformen sind beispielsweise Granulate, Pulver, Tabletten, Dragees, (Mikro)Kapseln, Zäpfchen, Sirupe, Emulsionen, Suspensionen, Tropfen oder injizierbare Lösungen sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Laktose, Mannit und andere Zucker, Talkum, Milcheiweiß, Gelatine, Stärke Cellulose und ihre Derivate, pflanzliche Öle, Polyethylenglykole und Lösungsmittel, wie etwa steriles Wasser, genannt.

Vorzugsweise werden die Präparate in Dosierungseinheiten hergestellt und verabreicht, wobei jede Einheit eine bestimmte Dosis an aktiver Substanz gemäß Formel I enthält. Bei festen Dosierungseinheiten wie Tabletten, Kapseln und Suppositorien, kann diese Dosis bis zu 1000 mg, bevorzugt jedoch 50 bis 300 mg, und bei Injektionslösungen in Ampullenform bis zu 200 mg, vorzugsweise aber 20 bis 100 mg, betragen.

Für die Behandlung eines an entzündlichen Prozessen leidenden erwachsenen Patienten sind - je nach Wirksamkeit der Verbindungen gemäß Formel I am Menschen - Tagesdosen von 100 bis 500 mg Wirkstoff, vorzugsweise 200 bis 300 mg, bei oraler Verabreichung und von 20 bis 150 mg, bevorzugt 40 bis 80 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilter Dosen

in bestimmten Intervallen erfolgen.

Die Erfindung wird durch die nachfolgenden Beispiele erläutert.

0090353

Beispiel 1a

DL-2-(6-Phenylpyridin-2-oxy)-propansäuremethylester

34,2 g 6-Phenylpyridon-2 werden in 200 ml Dimethylformamid gelöst, nacheinander mit 24,5 g DL-α-Chlorpropionsäuremethylester und 27,3 g $K_2CO_3$ versetzt und anschließend bei 90°C 8 Stunden gerührt. Danach wird die Reaktionsmischung heiß filtriert und der Filterkuchen mit etwas Aceton gewaschen. Das Aceton und das Lösemittel werden unter vermindertem Druck (bis zu 13 mbar) abdestilliert und der Rückstand über eine 20 cm Vigreux-Kolonne bei stark vermindertem Druck destilliert. Man erhält 44,3 g vom Kp 177°C bei 3 mbar als farbloses Öl (86 % d.Th.). Bei gleicher Arbeitsweise wie vorstehend beschrieben können auch Dimethylsulfoxid, Dimethylacetamid, N-Methylpyrrolidon-2 oder Dimethyldiglykol als Lösungsmittel und Natriumcarbonat als Base verwendet werden.

Beispiel 1b

gemäß Beispiel 1a, jedoch unter Verwendung äquivalenter Mengen der entsprechenden Verbindung der Formel II und III (DL-, D- oder L-Form) werden die folgenden, in Tabelle 1 aufgeführten Verbindungen der Formel I hergestellt.

Tabelle 1:

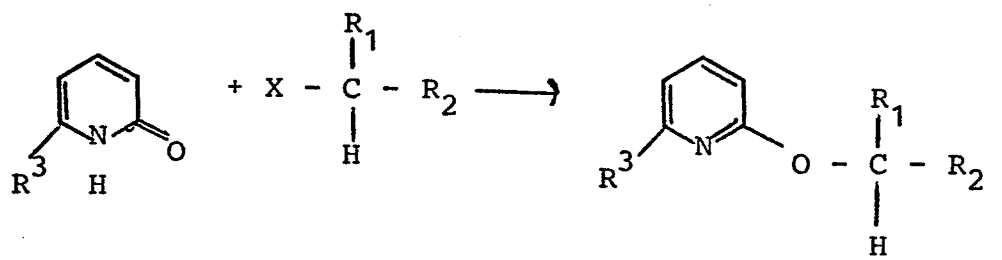

| Verb. Nr. | X | $R_3$ | $R_1$ | $R_2$ | |
|---|---|---|---|---|---|
| 1.1 | Cl | $C_6H_5$ | H | $-\overset{\overset{O}{\|}}{C}-O-CH_3$ | Fp.: 62°C<br>D,L |
| 1.2 | Br | $C_6H_5$ | $CH_3$ | $-\overset{\overset{O}{\|}}{C}-O-C_2H_5$ | $Kp_{2,5}$=175-177°C<br>D,L |
| 1.3 | Br | $C_6H_5$ | $CH_3$ | $-\overset{\overset{O}{\|}}{C}-O-CH(CH_3)_2$ | Fp.: 74°C<br>D,L |
| 1.4 | Cl | $C_6H_5$ | $CH_3$ | $-\overset{\overset{O}{\|}}{C}-O-(CH_2)_{11}-CH_3$ | Fp.: 39-40°C<br>D,L |
| 1.5 | Cl | $C_6H_5$ | $CH_3$ | $-\overset{\overset{O}{\|}}{C}-O-CH_2-C_6H_5$ | Fp.: 67-68°C<br>D,L |
| 1.6 | Br | $C_6H_5$ | $C_2H_5$ | $-\overset{\overset{O}{\|}}{C}-O-CH_3$ | |
| 1.7 | Br | $C_6H_5$ | $C_2H_5$ | $-\overset{\overset{O}{\|}}{C}-O-C_2H_5$ | $Kp_2$ = 182°C<br>D,L |
| 1.8 | Br | $C_6H_5$ | $-(CH_2)_2-CH_3$ | $-\overset{\overset{O}{\|}}{C}-O-CH_3$ | |
| 1.9 | Br | $C_6H_5$ | $-(CH_2)_2-CH_3$ | $-\overset{\overset{O}{\|}}{C}-O-C_2H_5$ | $Kp_2$ = 185°C<br>D,L |

0090353

Tabelle 1 Fortsetzung

| Verb. Nr. | X | $R_3$ | $R_1$ | $R_2$ | |
|---|---|---|---|---|---|
| 1.10 | Br | $C_6H_5$ | $-CH(CH_3)_2$ | $-\overset{O}{\overset{\|}{C}}-O-CH_3$ | |
| 1.11 | 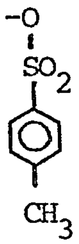 | $C_6H_5$ | H | $-CH_2-O-(CH_2)_3-CH_3$ | |
| 1.12 | 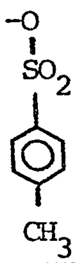 | $C_6H_5$ | H | $-CH_2-O-CH_3$ | $Kp_{0,4}$ = 128-131°C D,L |
| 1.13 | Br | 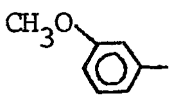 | $CH_3$ | $-\overset{O}{\overset{\|}{C}}-O-C_2H_5$ | $Kp_{0,5}$ = 178-180°C D,L |
| 1.14 | Br | 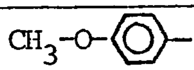 | $CH_3$ | $-\overset{O}{\overset{\|}{C}}-O-C_2H_5$ | |
| 1.15 | Br | 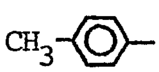 | $CH_3$ | $-\overset{O}{\overset{\|}{C}}-O-C_2H_5$ | $Kp_{0,6}$ = 145°C D,L |
| 1.16 | Br | 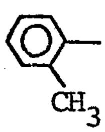 | $CH_3$ | $-\overset{O}{\overset{\|}{C}}-O-C_2H_5$ | |
| 1.17 | Br | 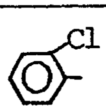 | $CH_3$ | $-\overset{O}{\overset{\|}{C}}-O-CH_3$ | |
| 1.18 | Br | 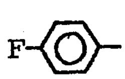 | $CH_3$ | $-\overset{O}{\overset{\|}{C}}-O-C_2H_5$ | $Kp_{0,1}$ = 125-127°C D,L |

Tabelle 1 Fortsetzung

| Verb. Nr. | X | $R_3$ | $R_1$ | $R_2$ | |
|---|---|---|---|---|---|
| 1.19 | Br | 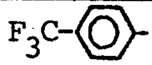 | $CH_3$ | 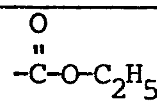 | |
| 1.20 | Br | 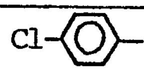 | $CH_3$ | 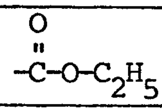 | $Kp_{0,5}$ 155-160°C D,L |
| 1.21 | Br | 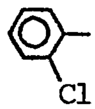 | $CH_3$ | 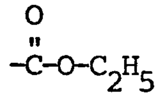 | |
| 1.22 | Br | 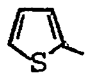 | $CH_3$ | 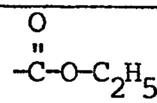 | |
| 1.23 | Br | 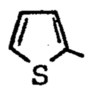 | $CH_3$ | 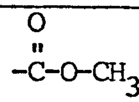 | |
| 1.24 | Br | 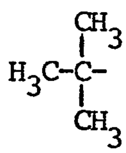 | $CH_3$ | 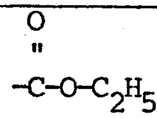 | |
| 1.25 D(+) | Cl | $C_6H_5$ | $CH_3$ | 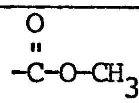 | $Kp_{0,8}$ 150-154°C $[\alpha]_D^{25}$ = +11,4° ($CHCl_3$) |
| 1.26 | Cl | 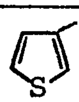 | $CH_3$ | 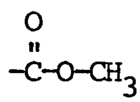 | |
| 1.27 | Cl | 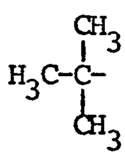 | $CH_3$ | 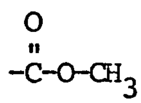 | |

Tabelle 1 Fortsetzung

| Verb. Nr. | X | $R_3$ | $R_1$ | $R_2$ | |
|---|---|---|---|---|---|
| 1.28 | Cl | 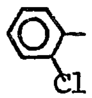 | $CH_3$ | $-\overset{O}{\overset{\|}{C}}-O-CH_3$ | |
| 1.29 | Cl | 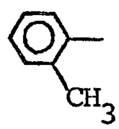 | $CH_3$ | $-\overset{O}{\overset{\|}{C}}-O-CH_3$ | |
| 1.30 D(+) | Cl | 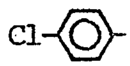 | $CH_3$ | $-\overset{O}{\overset{\|}{C}}-OCH_3$ | $Kp_{0,6}$ = 150-155°C $[\alpha]_D^{25}$ = + 11,4° ($CHCl_3$) |
| 1.31 D(+) | Cl | 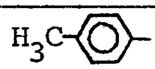 | $CH_3$ | $-\overset{O}{\overset{\|}{C}}-OCH_3$ | Fp.: 102°C $[\alpha]_D^{25}$ = +14,3° ($CHCl_3$) |
| 1.32 D(+) | Cl | 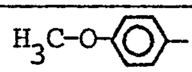 | $CH_3$ | $-\overset{O}{\overset{\|}{C}}-OCH_3$ | Fp.: 125-126 $[\alpha]_D^{25}$ = +14,0° ($CHCl_3$) |
| 1.33 D(+) | Cl | 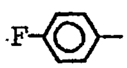 | $CH_3$ | $-\overset{O}{\overset{\|}{C}}-OCH_3$ | $Kp_{0.1}$ = 127-135°C $[\alpha]_D^{25}$ = + 11,2° ($CHCl_3$) |
| 1.34 D(+) | Cl | 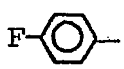 | $CH_3$ | $-\overset{O}{\overset{\|}{C}}-OC_2H_5$ | $Kp_{0,2}$ = 145-150°C $[\alpha]_D^{25}$ = +11,4° ($CHCl_3$) |
| 1.35 L(-) | Cl | 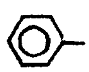 | $CH_3$ | $-\overset{O}{\overset{\|}{C}}-OC_2H_5$ | $Kp_{0.6}$ = 150-152 $[\alpha]_D^{25}$ = -12,1° ($CHCl_3$) |
| 1.36 | Cl | 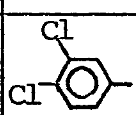 | $CH_3$ | $-\overset{O}{\overset{\|}{C}}-OC_2H_5$ | Fp.: 70°C D,L |

Beispiel 2

DL-2-[6-(3,4-Dichlorphenyl)-pyridin-2-oxy]-propansäure-ethylester

Zur Suspension von 3 g Natriumhydrid (80 %ige Öldispersion) in 200 ml Dimethylformamid gibt man unter Rühren und Kühlen 24 g 6-(3,4-Dichlorphenyl)-pyridon-2 portionsweise so zu, daß die Temperatur 30°C nicht übersteigt, rührt bei 40°C nach bis die Wasserstoffentwicklung beendet ist, tropft anschließend 18,1 g DL-$\alpha$-Brompropionsäureethylester zu und rührt 5 h bei 80°C nach. Nach dem Abkühlen wird die Reaktionsmischung in 500 ml Eiswasser gegossen, mit Methylenchlorid extrahiert, wie üblich aufgearbeitet und der Rückstand unter stark vermindertem Druck destilliert. Man erhält 25,5 g vom Kp 180°C bei 0,3 mbar, vom Fp.: 70°C (75 % d. Th.).

Beispiel 3

DL-2-(6-Phenylpyridin-2-oxy)-ethansäure

Zur Lösung von 17,1 g 6-Phenylpyridon-2 in 100 ml Dimethylformamid gibt man 0,3 g Tetrabutylammoniumbromid und unter intensivem Rühren 12,3 g feinstgepulverte KOH. Anschließend tropft man bei 80°C 12,2 g Chloressigsäureethylester zu, rührt intensiv 4 h nach, versetzt mit 10 ml Wasser, rührt weitere 30 min. nach und zieht unter vermindertem Druck das Lösungsmittel ab. Der Rückstand wird in 40 ml Wasser aufgenommen, die Lösung mit 2 n $H_2SO_4$ angesäuert, die ausgefallene Säure abgesaugt, im Trockenschrank bei 50°C getrocknet und dann aus Diisopropylether/Hexan umkristallisiert. Man erhält 14,9 g vom Fp. 125-126°C.

Beispiel 4

DL-2-(6-Phenylpyridin-2-oxy)-propansäure

Eine Mischung aus 12,9 g DL-2-(6-Phenylpyridin-2-oxy)-propansäuremethylester 2,4 g NaOH, 10 ml Methanol werden 30 min auf 80°C erhitzt. Nach dem Abkühlen wird mit 2 n $H_2SO_4$ angesäuert, die ausgefallene Säure im Trockenschrank bei 50°C getrocknet und dann aus Diisopropylether / Hexan umkristallisiert. Man erhält 10,9 g vom Fp.: 142-143°C (90 % d.Th.).

Beispiel 5

gemäß Beispiel 4, jedoch unter Verwendung entsprechender Mengen von Verbindungen aus Tabelle 1 und NaOH werden die folgenden, in Tabelle 2 aufgeführten Verbindungen der Formel I hergestellt.

0090353

Tabelle 2:

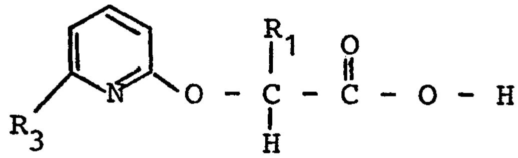

| Verb. Nr. | $R_3$ | $R_1$ | hergestellt aus Verb.Nr. | Fp.: [°C] | $[\alpha]_D^{25}$ ($CH_3OH$) |
|---|---|---|---|---|---|
| 2.1 | $C_6H_5$ | $-C_2H_5$ | 1.7 | | 0° |
| 2.2 | 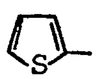 | $CH_3$ | 1.22 | 154 | 0° |
| 2.3 | 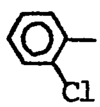 | $CH_3$ | 1.21 | | 0° |
| 2.4 | 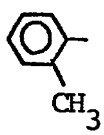 | $CH_3$ | 1.16 | | 0° |
| 2.5 | 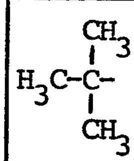 | $CH_3$ | 1.24 | | 0° |
| 2.6 | 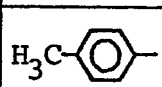 | $CH_3$ | 1.15 | 169 | 0° |
| 2.7 | 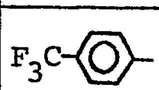 | $CH_3$ | 1.19 | | 0° |
| 2.8 D(+) | 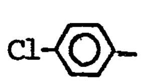 | $CH_3$ | 1.30 | 156-157 | + 7,0° |

Tabelle 2 (Fortsetzung)

| Verb. Nr. | $R_3$ | $R_1$ | hergestellt aus Verb.Nr. | Fp.: [°C] | $[\alpha]_D^{25}$ ($CH_3OH$) |
|---|---|---|---|---|---|
| 2.9 D(+) | 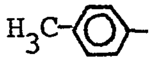 | $CH_3$ | 1.31 | 169-170 | + 7,2° |
| 2.10 D(+) | 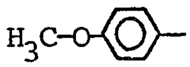 | $CH_3$ | 1.32 | 160 | + 11,0° |
| 2.11 D(+) | 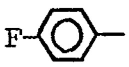 | $CH_3$ | 1.33 | 118-119 | + 9,4° |
| 2.12 L(-) | 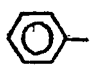 | $CH_3$ | 1.35 | 125-126 | -10,0° |

Beispiel 6

D(+)-2-(6-Phenylpyridin-2-oxy)-propansäure

25,7 g D(+)-2-(6-Phenylpyridin-2-oxy)-propansäuremethylester, 20 ml Methanol, 20 g $H_2O$ und 4,8 g NaOH werden 3 Stunden bei 75°C gerührt und nach dem Abkühlen mit 2 n $H_2SO_4$ angesäuert. Die ausgefallene Säure wird auf dem Tonteller abgedrückt, in Diisopropylether gelöst und die Lösung mit $Na_2SO_4$ getrocknet. Nach dem Filtrieren gibt man in der Hitze bis zur Trübung Hexan zu. Beim Abkühlen kristallisieren 18,5 g vom Fp.: 126-126°C aus (76 % d.Th.), mit einem Drehwert $[\alpha]_D^{25}$ = + 9,4° ($CH_3OH$).

Beispiel 7

D(+)-2-(6-Phenylpyridin-2-oxy)-propansäureethylester

12,2 g D(+)-2-(6-Phenylpyridin-2-oxy)-propansäure werden im Gemisch aus 50 ml Toluol und 30 ml Ethanol 5 h am Rückfluß gekocht, das Lösungsmittelgemisch abdestilliert und der Rückstand unter stark vermindertem Druck fraktioniert. Man erhält 11,1 g vom Kp 138-142°C bei 0,5 mbar (82 % d.Th.), mit einem Drehwert $[\alpha]_D^{20}$ = 10,0° ($CHCl_3$).

Beispiel 8

6-Phenylpyridin-2-oxyethyl-ethylether

Die Suspension aus 8,6 g 6-Phenylpyridon-2, 0,5 g Tetrabutylammoniumbromid, 5,4 g 2-Chlordiethylether, 100 ml Toluol und 20 ml 50%ige Natronlauge wird 5 h bei 100°C intensiv gerührt. Nach dem Abkühlen versetzt man mit 30 ml Wasser, trennt die organische Phase ab, wäscht mit 2 n NaOH nach, trocknet über $Na_2SO_4$, zieht unter vermindertem Druck das Lösungsmittel ab und destilliert den Rückstand unter stark vermindertem Druck. Man erhält 7,9 g farbloses Öl (66 % d.Th.).

Beispiel 9

DL-2-(6-Phenylpyridin-2-oxy)-propanol

Zur Suspension von 2,05 g Lithiumaluminiumhydrid in 75 ml absolutem Diethylether tropft man unter Rühren bei Raumtemperatur die Lösung von 13,6 g DL-2-(6-Phenylpyridin-2-oxy)-propansäureethylester in 30 ml absolutem Diethylether und rührt 1 h am Rückfluß. Nach dem Abkühlen wird mit Wasser hydrolisiert, mit 10%iger Schwefelsäure angesäuert und die wässrige Phase mit NaCl gesättigt, abgetrennt, mit Äther extrahiert, die vereinigten organischen Phasen über $Na_2SO_4$ getrocknet und das Lösungsmittel unter vermindertem Druck abgezogen. Der Rückstand wird wie im Beispiel 1a beschrieben destilliert. Man erhält 10,9 g vom Kp 181°C bei 6-7 mbar (95 % d.Th.).

Beispiel 10

D(+)-2-(6-Phenylpyridin-2-oxy)-propanol

Gemäß Beispiel 9, jedoch unter Verwendung von 12,9 g D(+)-2-(6-Phenylpyridin-2-oxy)-propansäuremethylester erhält man 11,0 g vom Kp 180°C bei 6 mbar (96 % d.Th.), mit einem Drehwert $[\alpha]_D^{20} = + 2,1^\circ$ ($CHCl_3$).

Beispiel 11

gemäß Beispiel 9, jedoch unter Verwendung entsprechender Mengen von Verbindungen aus Tabelle 1 und Lithiumaluminiumhydrid, werden die folgenden, in Tabelle 3 aufgeführten Verbindungen der Formel I hergestellt:

Tabelle 3

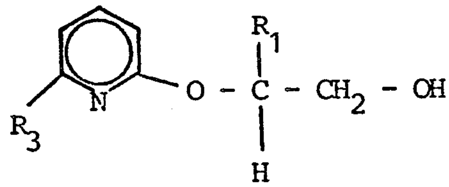

| Verb. Nr. | $R_3$ | $R_1$ | hergestellt aus Verb.-Nr. | |
|---|---|---|---|---|
| 3.1 | $C_6H_5$ | $-C_2H_5$ | 1.7 | |
| 3.2 | 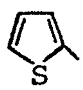 | $CH_3$ | 1.22 | |
| 3.3 | 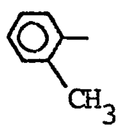 | $CH_3$ | 1.16 | |
| 3.4 | 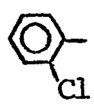 | $CH_3$ | 1.21 | |
| 3.5 | 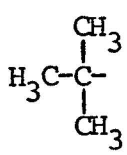 | $CH_3$ | 1.24 | |
| 3.6 | 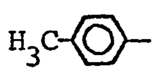 | $CH_3$ | 1.15 | $Kp_{0,1}$=155°C<br>Fp.: 55°C<br>D,L |
| 3.7 | 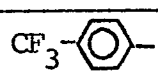 | $CH_3$ | 1.19 | |
| 3.8 | 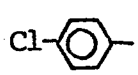 | $CH_3$ | 1.20 | $Kp_{0,5}$= 175°C<br>Fp.: 61°C<br>D,L |
| 3.9 | 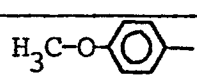 | $CH_3$ | 1.14 | $Kp_{0,1}$= 175°C<br>Fp.: 58°C<br>D,L |

Tabelle 3 (Fortsetzung):

| Verb. Nr. | $R_3$ | $R_1$ | hergestellt aus Verb.-Nr. | |
|---|---|---|---|---|
| 3.10 D(+) | 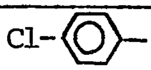 | $CH_3$ | 1.30 | Fp.: 65°C<br>$[\alpha]_D^{25}$ = +1,7° ($CHCl_3$) |
| 3.11 D(+) | 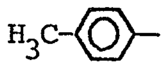 | $CH_3$ | 1.31 | Fp.: 55°C<br>$[\alpha]_D^{25}$ = + 2,4° ($CHCl_3$) |
| 3.12 D(+) | 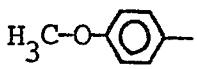 | $CH_3$ | 1.32 | Fp.: 58°C<br>$[\alpha]_D^{25}$ = + 2,4° ($CHCl_3$) |
| 3.13 | 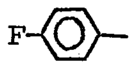 | $CH_3$ | 1.18 | $Kp_{0,1}$ = 150°C<br>D,L |
| 3.14 D(+) | 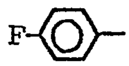 | $CH_3$ | 1.33 | $Kp_{0,1}$ = 150°C<br>$[\alpha]_D^{25}$ = + 1,7° ($CHCl_3$) |
| 3.15 L(-) | 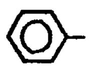 | $CH_3$ | 1.35 | $Kp_{0,6}$ = 165°C<br>$[\alpha]_D^{25}$ = -2,2° ($CHCl_3$) |

Beispiel 12

DL-2-(6-Phenylpyridin-2-oxy)-propylacetat

Zur Lösung von 4,6 g DL-2-(6-Phenylpyridin-2-oxy)-propanol (aus Beispiel 9) in 10 ml Pyridin tropft man unter Rühren bei Raumtemperatur 2,3 g Acetanhydrid, rührt bei 60°C 4 h nach, läßt abkühlen, gießt in 50 ml Eiswasser, extrahiert mit $CH_2Cl_2$ und arbeitet wie üblich auf. Der Rückstand wird unter stark vermindertem Druck destilliert. Man erhält 4,3 g farbloses Öl (80 % d.Th.) vom Kp. 153-155°C bei 0,3 mbar.

Beispiel 13

gemäß Beispiel 12, jedoch unter Verwendung entsprechender Mengen von Verbindungen aus Tabelle 3 und Acetanhydrid werden die folgenden, in Tabelle 4 aufgeführten Verbindungen der Formel I hergestellt.

Tabelle 4

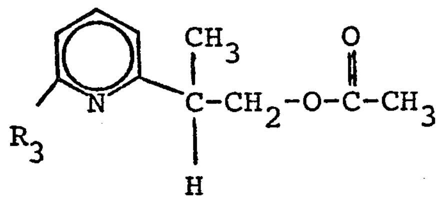

| Verb. Nr. | $R_3$ | hergestellt aus Verb.Nr. | |
|---|---|---|---|
| 4.1 | 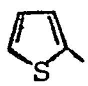 | 3.2 | |
| 4.2 | 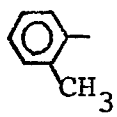 | 3.3 | |
| 4.3 | 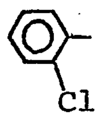 | 3.4 | |
| 4.4 | 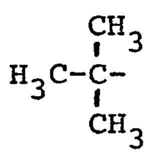 | 3.5 | |
| 4.5 | 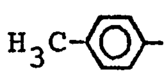 | 3.6 | |
| 4.6 | 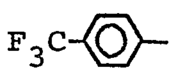 | 3.7 | |
| 4.7 | 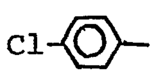 | 3.8 | |
| 4.8 | 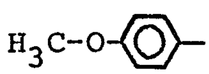 | 3.9 | |
| 4.9 D(+) | 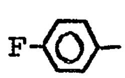 | 3.14 | |

Beispiel 14

DL-2-(6-Phenylpyridin-2-oxy)-propyl-iso-butyrat

Zur Lösung von 4,6 g DL-2-(6-Phenylpyridin-2-oxy)-propanol (aus Beispiel 9) in 20 ml Methylenchlorid und 2,2 g Triethylamin tropft man unter Rühren und Kühlen bei 0°C die Lösung von 2,1 g iso-Buttersäurechlorid in 5 ml Methylenchlorid, läßt auf Raumtemperatur aufwärmen und rührt dann 2 h am Rückfluß nach. Nach dem Abkühlen wird mit Wasser ausgeschüttelt, wie üblich aufgearbeitet und der Rückstand unter starm vermindertem Druck destilliert. Man erhält 4 g (67 % d.Th.) vom Kp.: 155-158°C bei 0,2 mbar.

Beispiel 15

Gemäß Beispiel 14, jedoch unter Verwendung äquivalenter Mengen von Verbindungen aus Beispiel 9, 10 oder Tabell 3 und eines entsprechenden Acylchlorides werden folgende, in Tabelle 5 aufgeführten Verbindungen der Formel I hergestellt:

Tabelle 5

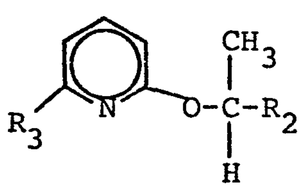

| Verb. Nr. | $R_2$ | $R_3$ | herge-stellt aus Verb. | |
|---|---|---|---|---|
| 5.1 | 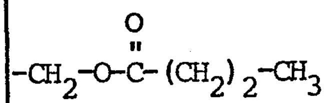 | 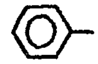 | Beisp. 9 | $Kp_{0,2}$=155-158°C<br>D,L |
| 5.2 | 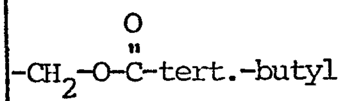 | 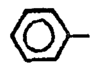 | Beisp. 9 | $Kp_{0,5}$= 160-165°C<br>D,L |
| 5.3 | 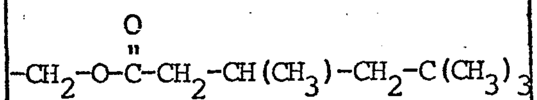 | 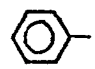 | Beisp. 9 | $Kp_{0,6}$=175-180°C<br>D,L |
| 5.4 | 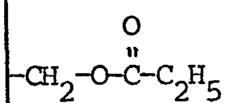 | 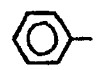 | Beisp. 9 | $Kp_{0,3}$=165-170°C<br>D,L |
| 5.5 | 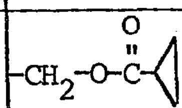 | 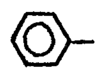 | Beisp. 9 | $Kp_{0,5}$=170-175°C<br>D,L |
| 5.6 | 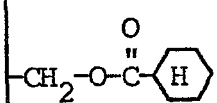 | 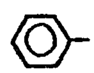 | Beisp. 9 | |
| 5.7 | 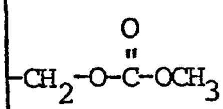 | 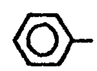 | Beisp. 9 | Fp.:62-64°C<br>D,L |
| 5.8 | 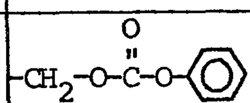 | 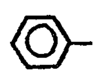 | Beisp. 9 | Fp.:68-70°C<br>D,L |
| 5.9 | 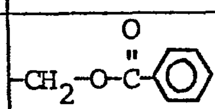 | 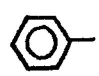 | Beisp. 9 | Öl<br>D,L |
| 5.10 | 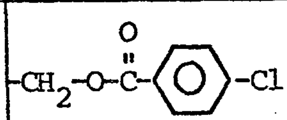 | 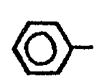 | Beisp. 9 | Fp.:68-69°C<br>D,L |
| 5.11 | 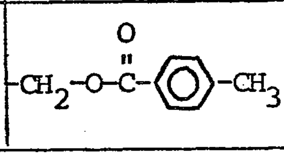 | 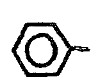 | Beisp. 9 | |

Fortsetzung Tabelle 5

| Verb. Nr. | $R_2$ | $R_3$ | herge-stellt aus Verb.(Nr.) | |
|---|---|---|---|---|
| 5.12 | 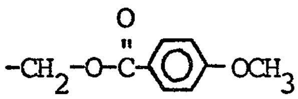 | 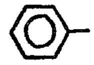 | Beisp. 9 | |
| 5.13 | 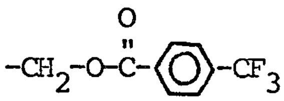 | 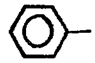 | Beisp. 9 | |
| 5.14 | 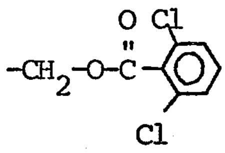 | 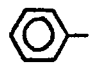 | Beisp. 9 | |
| 5.15 D(+) | O<br>"<br>$-CH_2-O-C-CH_3$ | 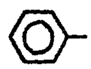 | Beisp. 10 | $Kp_{0,3}$=153-155°C<br>$[\alpha]_D^{25}$ = +3,4°<br>($CHCl_3$) |
| 5.16 D(+) | O<br>"<br>$-CH_2-O-C-C_2H_5$ | 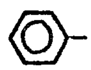 | Beisp. 10 | $Kp_{0,2}$= 165-168°C<br>$[\alpha]_D^{25}$ =+3,5°<br>($CHCl_3$) |
| 5.17 D(+) | O<br>"<br>$-CH_2-O-C-C_3H_7$ | 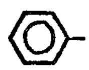 | Beisp. 10 | $Kp_{0,2}$= 155-158°C<br>$[\alpha]_D^{25}$ = +3,2°<br>($CHCl_3$) |
| 5.18 D(+) | O<br>"<br>$-CH_2-O-C-CH(CH_3)_2$ | 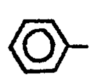 | Beisp. 10 | $Kp_{0,2}$=155-158°C<br>$[\alpha]_D^{25}$ = + 3,3°<br>($CHCl_3$) |
| 5.19 D(+) | O<br>"<br>$-CH_2O-C-CH_3$ | 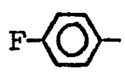 | 3.14 | |
| 5.20 D(+) | O<br>"<br>$-CH_2-O-C-C_2H_5$ | 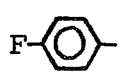 | 3.14 | |
| 5.21 D(+) | O<br>"<br>$-CH_2-O-C-CH(CH_3)_2$ | 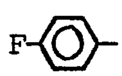 | 3.14 | |

Fortsetzung Tabelle 5:

| Verb. Nr. | $R_2$ | $R_3$ | hergestellt aus Verb.Nr. | |
|---|---|---|---|---|
| 5.22 D(+) | $-CH_2O-\overset{O}{\overset{\|\|}{C}}-C_3H_7$ | 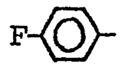 | 3.14 | |
| 5.23 D(+) | 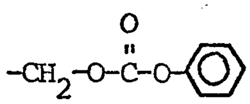 | 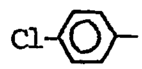 | 3.10 | Fp.: 75-76$^{\circ}$C $[\alpha]_D^{25}$ = + 6,0$^{\circ}$ ($CHCl_3$) |
| 5.24 D(+) | $-CH_2-O-\overset{O}{\overset{\|\|}{C}}-C_2H_5$ | 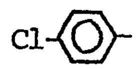 | 3.10 | $Kp_{0,1}$= 173-179$^{\circ}$C $[\alpha]_D^{25}$ =+ 3,5$^{\circ}$ $CHCl_3$) |

Beispiel 16

DL-2-(6-Phenylpyridin-2-oxy)-propyl-methylether

Zur Suspension von 0,6 g NaH (80 %ige Öldispersion) in 30 ml abs. Tetrahydrofuran (THF) tropft man unter Rühren bei 20°C die Lösung von 4,6 g DL-2-(6-Phenylpyridin-2-oxy)-propanol (aus Beispiel 9) in 5 ml abs. THF, rührt nach bis die Wasserstoffentwicklung beendet ist, kühlt auf 0°C ab, tropft die Lösung von 2,8 g Methyljodid zu, läßt auf Raumtemperatur aufwärmen und kocht anschließend 2 h am Rückfluß. Nach Abkühlung wird unter vermindertem Druck das Lösungsmittel abgezogen, wie üblich mit Wasser / Methylenchlorid aufgearbeitet und der Rückstand unter stark vermindertem Druck destilliert. Man erhält 3,9 g (80 % d.Th.) vom Kp. 128-131°C bei 0,5 mbar.

Beispiel 17

DL-2-(6-Phenylpyridin-2-oxy)-propyl-butylether

Gemäß Beispiel 16, jedoch unter Verwendung von 4,6 g DL-2-(6-Phenylpyridin-2-oxy)-propanol (aus Beispiel 9) und 2,7 g n-Butylbromid erhält man 4,3 g (75 % d.Th.).

Beispiel 18

DL-2-(6-Phenylpyridin-2-oxy)-propanal

Der Versuch wird unter Stickstoff als Inertgas durchgeführt. Zur Lösung von 2,32 ml Oxalylchlorid in 25 ml abs. Methylenchlorid tropft man unter Rühren bei -60°C die Lösung von 3,78 ml Dimethylsulfoxid in 10 ml abs. Methylenchlorid, rührt 5 min. nach und gibt anschließend die Lösung von 3,7 g DL-2-(6-Phenylpyridin-2-oxy)-propanol (aus Beispiel 9) in 20 ml abs. Methylenchlorid auf einmal

zu. Anschließend rührt man 20 min. bei -60°C nach, tropft 14,4 ml Triethylamin zu und läßt langsam auf Raumtemperatur aufwärmen, gibt dann 50 ml Wasser zu und arbeitet wie üblich auf. Der Rückstand wird in 40 ml Äther aufgenommen, über Celite filtriert und das Lösungsmittel unter vermindertem Druck abgezogen. Man erhält 4 g farbloses Öl (86 % d.Th.).

Beispiele für die Herstellung von Verbindungen der Formel II:

Beispiel 19

6-Phenylpyridon-2

a) 5-Phenyl-5-oxopentansäuremethylester

In einem 5 l-Autoklaven werden 3220 g Acetophenon, 484 g Methylacrylat, 80 g Isopropylamin und 2,7 g Benzoesäure drei Stunden lang auf 200°C erhitzt. Die anschließende Destillation unter vermindertem Druck ergab neben nichtumgesetztem Methylacrylat und Acetophenon 640 g vom Kp 175°C bei 7,5 mbar (55,5 % d.Th. bezogen auf Methylacrylat).

b) 6-Phenyl-3,4-dihydropyridon-2:

In einem 500 ml Kolben ausgerüstet mit absteigendem Kühler werden 300 g 5-Phenyl-5-oxo-pentansäuremethylester auf 180°C erhitzt. Dann wird drei Stunden lang Ammoniak eingeleitet. Dabei destillieren das entstehende Wasser und Methanol über den absteigenden Kühler ab. Nach dem Abkühlen erstarrt das Reaktionsgemisch zu einem Kristallbrei. Man saugt ab und erhält 130 g vom Fp. 158°C (53 % d.Th.). Die Mutterlauge enthält hauptsächlich nichtumgesetztes Ausgangsmaterial.

c) 6-Phenylpyridon-2

In einen 500 ml Glaskolben, ausgerüstet mit Rührer, Thermometer, absteigendem Kühler und Gasuhr, wurden 250 ml Methyl-butyl-diglykol, 5 g Katalysator (2 % Pd auf Kohle) auf 30 g 6-Phenyl-3,4-dihydro-pyridon-2 gegeben. Die Apparatur wurde mit Stickstoff gespült und anschließend das Methylbutyldiglykol bis zum Rückfluß erhitzt. Dabei entstanden während 5 Stunden 4,3 l Abgas, die zu 90 % aus Wasserstoff bestanden. Die Apparatur wurde wieder mit $N_2$ gespült; in der Hitze wurde die Lösung vom Katalysator abfiltriert. Beim Erkalten kristallisierten aus der Mutterlauge 25,3 g vom Fp. 198°C aus (82 % d.Th.).

Der Katalysator ist wiederverwendbar. Selbst nach zehnmaligem Einsatz beträgt die Ausbeute an 6-Phenylpyridon-2 ca. 80 %.

Beispiel 20

Gemäß Beispiel 19, jedoch unter Verwendung entsprechender Mengen eines Ketons der Formel $R_3-\overset{O}{\overset{\|}{C}}-CH_3$ und eines Acrylsäureesters der Formel

$$CH_2=CH-\overset{O}{\overset{\|}{C}}-O-C_2H_5$$

werden die folgenden, in Tabelle 6 aufgeführten Verbindungender Formel II hergestellt:

Tabelle 6

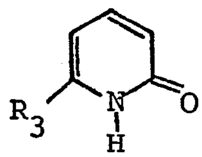

(II)

| Verb. Nr. | $R_3$ | Fp.:/¯°C_7 |
|---|---|---|
| 6.1 | 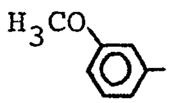 | |
| 6.2 | 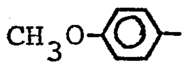 | 172-176 a) |
| 6.3 | 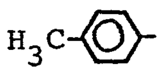 | 170-175 a) |
| 6.4 | 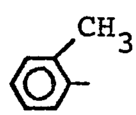 | |
| 6.5 | 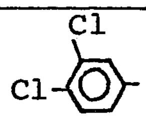 | |
| 6.6 | 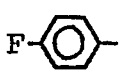 | 178-183 a) |
| 6.7 | 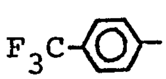 | |
| 6.8 | 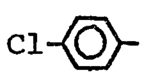 | 203-204 |

a) Schmelzpunkt durch geringe Verunreinigungen mit den entsprechenden 3,4,5,6-Tetrahydropyridin-2-onen unscharf.

Fortsetzung Tabelle 6

| Verb. Nr. | $R_3$ | Fp.: /‾°C_7 |
|---|---|---|
| 6.9 | 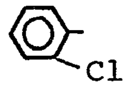 | |
| 6.10 | 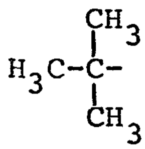 | |
| 6.11 | 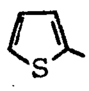 | 195 |

PATENTANSPRÜCHE

1. Pyridon-2-derivate der Formel I,

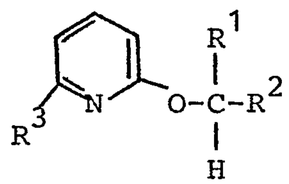

(I)

ihre Stereoisomeren und ihre Salze mit einer physiologisch verträglichen Säure oder Base, in der

$R^1$ Wasserstoff oder Alkyl mit 1 bis 6 Kohlenstoffatomen,

$R^2$ eine $-CH{=}O$, $-COOR^4$ oder $-CH_2-O-R^5$-Gruppe bedeutet, wobei

$R^4$ für Wasserstoff, Alkyl mit 1 bis 12 Kohlenstoffatomen oder Benzyl und

$R^5$ für Wasserstoff, Alkyl mit 1 bis 4 Kohlenstoffatomen, Alkanoyl mit 1 bis 9 Kohlenstoffatomen, Cycloalkanoyl mit 4 bis 7 Kohlenstoffatomen, Alkoxycarbonyl mit 2 bis 5 Kohlenstoffatomen, Phenoxycarbonyl oder eine Benzoylgruppe stehen, welche ein oder zwei Halogen-, Trifluormethyl- oder Alkyl- oder Alkoxy-Substituenten mit jeweils 1 bis 4 Kohlenstoffatomen tragen kann und

$R^3$ für 2-Thienyl, 3-Thienyl, tert.-Butyl oder eine Phenylgruppe steht, welche einen oder zwei Substituenten tragen kann, die Halogen, Trifluormethyl oder Alkyl oder Alkoxy mit jeweils 1 bis 4 Kohlenstoffatomen bedeuten.

2. Verfahren zur Herstellung einer Verbindung der Formel I in Anspruch 1, dadurch gekennzeichnet, daß man eine Verbindung der Formel II

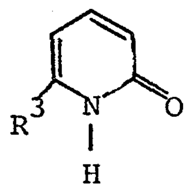

(II)

mit einer Verbindung der Formel III

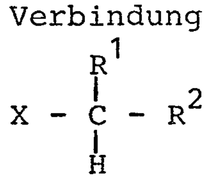

(III)

in denen $R^1$, $R^2$ und $R^3$ die im Anspruch 1 angegebenen Bedeutungen haben und X für Halogen, Alkylsulfonyloxy, substituiertes oder unsubstituiertes Arylsulfonyloxy oder den Rest eines aktiven Esters steht, umsetzt.

3. Verfahren nach Anspruch 2, dadurch gekennzeichnet, daß man zur Herstellung einer Verbindung der Formel I, in der $R^2$ -CH=O oder eine $-CH_2-O-R^5$-Gruppe ist, eine Verbindung der Formel I, in der $R^2$ eine $-COOR^4$-Gruppe ist, zur $CH_2OH$-Gruppe reduziert, diese dann zur -CH=O Gruppe oxidiert oder mit einem Alkylierungs- oder Acylierungsmittel zur $-CH_2-O-R^5$-Gruppe umsetzt.

4. Verfahren zur Herstellung der Verbindung II in Anspruch 2, dadurch gekennzeichnet, daß man eine Verbindung der Formel IV

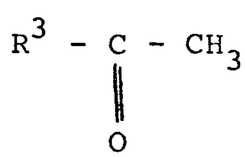

(IV)

mit einem Acrylsäureester der Formel V

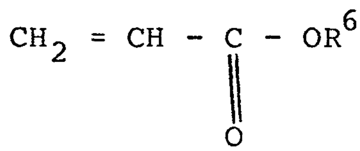

(V)

0090353
HOE 82/F 063

zu einer Verbindung der Formel VI

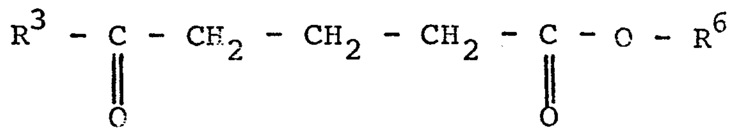 (VI)

umsetzt, diese dann mit Ammoniak zu einer Verbindung der Formel VII

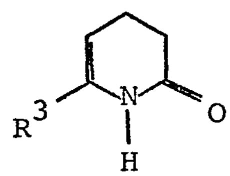 (VII)

zyklisiert und schließlich zu einer Verbindung der Formel II dehydriert, wobei in der vorstehenden Formel $R^3$ die in Anspruch 1 genannten Bedeutungen hat und $R^6$ ein Alkylrest mit 1 bis 6 Kohlenstoffatomen ist.

5. Arzneimittel, dadurch gekennzeichnet, daß es eine Verbindung der Formel I in Anspruch 1 enthält oder aus ihr besteht.

6. Verfahren zur Herstellung eines Arzneimittels gemäß Anspruch 5, dadurch gekennzeichnet, daß man eine Verbindung der Formel I oder eines ihrer Salze, gegebenenfalls zusammen mit einem üblichen pharmazeutischen Träger- und/oder Hilfsstoff, in eine für pharmazeutische Zwecke geeignete Darreichungsform bringt.

7. Verwendung einer Verbindung der Formel I von Anspruch 1 als Arzneimittel mit antiphlogistischer Wirkung.

PATENTANSPRÜCHE für Österreich:

1. Verfahren zur Herstellung von Pyridon-2-derivaten der Formel I,

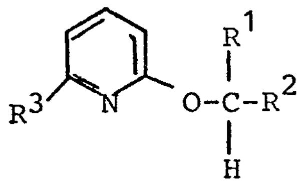

(I)

ihrer Stereoisomeren und ihrer Salze mit einer physiologisch verträglichen Säure oder Base, in der

$R^1$ Wasserstoff oder Alkyl mit 1 bis 6 Kohlenstoffatomen,

$R^2$ eine -CH=O, $-COOR^4$ oder $-CH_2-O-R^5$-Gruppe bedeutet, wobei

$R^4$ für Wasserstoff, Alkyl mit 1 bis 12 Kohlenstoffatomen oder Benzyl und

$R^5$ für Wasserstoff, Alkyl mit 1 bis 4 Kohlenstoffatomen, Alkanoyl mit 1 bis 9 Kohlenstoffatomen, Cycloalkanoyl mit 4 bis 7 Kohlenstoffatomen, Alkoxycarbonyl mit 2 bis 5 Kohlenstoffatomen, Phenoxycarbonyl oder eine Benzoylgruppe stehen, welche ein oder zwei Halogen-, Trifluormethyl- oder Alkyl- oder Alkoxy-Substituenten mit jeweils 1 bis 4 Kohlenstoffatomen tragen kann und

$R^3$ für 2-Thienyl, 3-Thienyl, tert.-Butyl oder eine Phenylgruppe steht, welche einen oder zwei Substituenten tragen kann, die Halogen, Trifluormethyl oder Alkyl oder Alkoxy mit jeweils 1 bis 4 Kohlenstoffatomen bedeuten,

dadurch gekennzeichnet, daß man eine Verbindung der Formel II

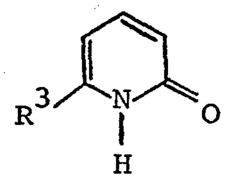

(II)

mit einer Verbindung der Formel III

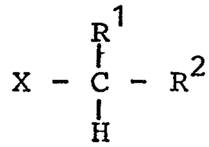

(III)

in denen $R^1$, $R^2$ und $R^3$ die vorstehend angegebenen Bedeutungen haben und X für Halogen, Alkylsulfonyloxy, substituiertes oder unsubstituiertes Arylsulfonyloxy oder den Rest eines aktiven Esters steht, umsetzt.

2. Verfahren nach Anspruch 1, dadurch gekennzeichnet, daß man zur Herstellung einer Verbindung der Formel I, in der $R^2$ $-CH=O$ oder eine $-CH_2-O-R^5$-Gruppe ist, eine Verbindung der Formel I, in der $R^2$ eine $-COOR^4$-Gruppe ist, zur $CH_2OH$-Gruppe reduziert, diese dann zur $-CH=O$ Gruppe oxidiert oder mit einem Alkylierungs- oder Acylierungsmittel zur $-CH_2-O-R^5$-Gruppe umsetzt.

3. Verfahren zur Herstellung der Verbindung II in Anspruch 1, dadurch gekennzeichnet, daß man eine Verbindung der Formel IV

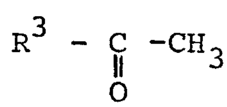

(IV)

mit einem Acrylsäureester der Formel V

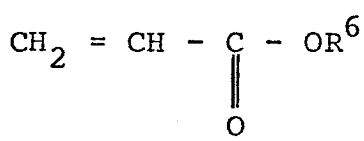

(V)

0090353
HOE 82/F 063

zu einer Verbindung der Formel VI

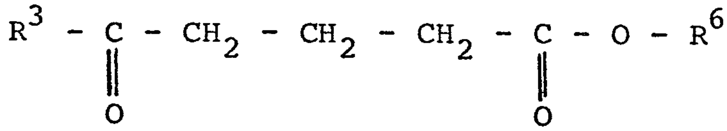

(VI)

umsetzt, diese mit Ammoniak zu einer Verbindung der Formel VII

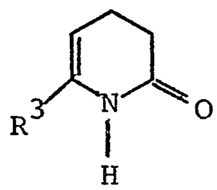

(VII)

zyklisiert und schließlich zu einer Verbindung der Formel II dehydriert, wobei in der vorstehenden Formel $R^3$ die genannten Bedeutungen hat und $R^6$ ein Alkylrest mit 1 bis 6 Kohlenstoffatomen ist.

Europäisches Patentamt

**EUROPÄISCHER RECHERCHENBERICHT**

0090353

Nummer der Anmeldung

EP 83 10 2926

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US-A-3 655 897 (B. WITZEL)<br>* Ansprüche 1,2 *<br>----- | 1,5 | C 07 D 213/64<br>A 61 K 31/44<br>C 07 D 409/04 //<br>(C 07 D 409/04<br>C 07 D 333/00<br>C 07 D 213/00 ) |
| | | | **RECHERCHIERTE SACHGEBIETE (Int. Cl. 3)** |
| | | | C 07 D 213/00<br>A 61 K 31/00<br>C 07 D 409/04 |

Der vorliegende Recherchenbericht wurde für alle Patentansprüche erstellt.

| Recherchenort | Abschlußdatum der Recherche | Prüfer |
|---|---|---|
| DEN HAAG | 16-08-1983 | VERHULST W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82